# FASCICULE DE BREVET EUROPEEN

(11) **EP 0 561 695 B1**
(45) Date de publication et mention de la délivrance du brevet: **06.05.1998**
(21) Numéro de dépôt: 93400677.6
(22) Date de dépôt: 17.03.1993
(51) Int. Cl.: A61K 9/00

(54) **Insert ophtalmique bioadhésif**
Bioadhäsive ophthalmologische Einsatz
Bioadhesive opthalmic insert

(30) Priorité: 20.03.1992 FR 9203390
(43) Date de publication de la demande: 22.09.1993
(73) Titulaire: VETOQUINOL S.A., F-70200 Lure (FR)
(72) Inventeur: Gurtler, Florian, CH-1224 Chene Bougeries (CH); Gurny, Robert, CH-1204 Geneve (CH)
(74) Mandataire: Kédinger, Jean-Paul

(56) Documents cités:
- EP-A- 0 077 261
- EP-A- 0 246 653
- EP-A- 0 316 838
- FR-A- 2 305 173
- FR-A- 2 319 375
- STN FILE SERVER & FILE CA & CHEMICAL ABSTRACTS, vol. 112, no. 8, résumé no. 62641z, Columbus, Ohio, US; & JP-A-01 071 822 (ROHTO PHARMACEUTICAL CO., LTD) 16-03-1989

## Description

L'invention a pour objet un insert ophtalmique bioadhésif, plus particulièrement un insert ophtalmique bioadhésif destiné à la libération prolongée et contrôlée d'une ou plusieurs substances médicamenteuses.

En médecine humaine comme en médecine vétérinaire, on connaît à ce jour de nombreuses compositions pharmaceutiques utilisées dans le traitement de troubles oculaires les plus variés, qu'il s'agisse d'inflammations telles que les conjonctivites, d'infections d'origine virale ou bactérienne, du glaucome ou du syndrôme de l'oeil sec (dry eye syndrom) par exemple. Ces compositions se présentent le plus souvent sous forme de collyres, de gels, de pommades, de films ou encore d'inserts que l'on dépose, dans ce dernier cas, soit dans le cul-de-sac conjonctival soit sous la paupière supérieure.

L'emploi d'inserts ophtalmiques est notamment recherché lorsque le traitement prescrit doit être de longue durée ou nécessite un temps de contact prolongé du principe actif, ou encore lorsque des applications doivent être répétées fréquemment. Dans de tel cas, on vise aussi bien le confort du sujet traité, par la réduction du nombre d'interventions nécessaires par exemple, que le relargage contrôlé de la substance active sur une période suffisamment longue. Pour l'essentiel, de tels inserts sont obtenus à partir d'un polymère ou copolymère hydrosoluble, notamment à partir d'un dérivé cellulosique hydrosoluble incorporant la substance active en proportions variables (voir p. ex. US-A-4.179.497, 4.343.787, 3.870.791 ou EP-A-0.108.661). On utilise alors un matériau polymère unique, que l'on s'efforce d'adapter au but visé en fonction de ses caractéristiques propres, en agissant sur des paramètres tels que viscosité, poids moléculaire, cristallinité par exemple. Les moyens d'action disponibles sont cependant fort limités.

A l'heure actuelle, les inserts ophtalmiques disponibles ne permettent pas d'obtenir des temps de séjour prolongés, tout en garantissant un relargage approprié de la substance active. De plus, on observe souvent un déplacement fortuit de l'insert, soit que celui-ci passe derrière l'oeil, soit qu'il sorte de l'orbite. L'invention a le mérite de proposer un nouveau type d'insert ophtalmique permettant de surmonter avantageusement de tels inconvénients, assurant notamment la permanence de son positionnement et le relargage durable du principe actif médicamenteux.

A titre d'état antérieur de la technique on citera en particulier :
- STN FILE SERVER and FILE CA and CHEMICAL ABSTRACTS, vol. 112, n° 8, résumé n° 62 641z, Colombus Ohio, US, et JP-A-01 071 822, qui décrit une formulation ophtalmique comprenant un principe actif sous forme de particules enrobées d'un polymère acrylique pratiquement insoluble dans l'eau, ces particules étant dispersées dans une masse d'hydroxypropylcellulose soluble dans l'eau;
- EP-A-0 316 838 qui décrit un insert ayant une structure multicouche ; la première couche est une matrice (a) contenant une substance active avec un polymère hydrosoluble ; la seconde couche (b) est appliquée sur une partie seulement de la surface de la première couche et est réalisée en un polymère non hydrosoluble ; la troisième couche (c) est facultative et réalisée en un polymère bioadhésif;
- EP-A-0 077 261 concerne un insert soluble essentiellement constitué de polymères hydrosolubles (alcool polyvinylique et hydroxypropylcellulose) et qui ne contient ni polymère bioadhésif, ni polymère non hydrosoluble.
- FR-A-2 305 173 concerne un insert insoluble dans le liquide lacrymal et donc insoluble dans l'eau ;
- FR-A-2 319 375 décrit un insert exempt de tout principe actif et préparé à partir d'un polymère hydrosoluble.

L'invention a pour objet un insert ophtalmique bioadhésif, en particulier un insert ophtalmique bioadhésif destiné à la libération prolongée et contrôlée d'au moins une substance médicamenteuse, qui se compose d'une matrice en matériau polymère composite dans laquelle est incorporée la substance médicamenteuse, caractérisé en ce que la dite matrice en matériau composite polymère composit comprend, en mélange :
- un polymère biocompatible hydrosoluble et
- un polymère biocompatible bioadhésif, ces deux polymères étant thermoformables ou extrudables.

L'invention a aussi pour objet un insert ophtalmique tel que défini ci-dessus, caractérisé en ce que ladite matrice en matériau polymère composite comprend, en mélange:
- un polymère biocompatible non hydrosoluble,
- un polymère biocompatible hydrosoluble et
- un polymère biocompatible bioadhésif.

Ces trois polymères étant thermoformables ou extrudables.

Avantageusement, la matrice en matériau polymère composite de l'insert selon l'invention, comprend de 50 à 99,5 % en poids du polymère biocompatible hydrosoluble et de 0,5 à 5,0 % en poids du polymère biocompatible bioadhésif, le complément éventuel à 100 % en poids étant constitué par le polymère biocompatible non-hydrosoluble.

Selon l'invention, on peut utiliser avantageusement à titre de polymère biocompatible hydrosoluble, une hydroxyalkylcellulose, une maltodextrine, un chitosan, un amidon modifié tel par exemple un amidon prégélatinisé, un amidon destructuré (voir par exemple US-A-4.900.361) ou un amidon partiellement hydrolysé, ou encore un alcool polyvinylique, cette énumération n'étant cependant pas exhaustive. On utilise de préférence une hydroxyalkylcellulose telle une hydroxyéthylcellulose ou une hydroxypropylcellulose de poids moléculaire compris entre 10'000 et 1'000'000 ou plus, préférablement compris entre 80'000 et 125'000. De tels produits se trouvent dans le commerce spécialisé.

Selon l'invention, à titre de polymère biocompatible non hydrosoluble, on peut avantageusement utiliser une alkylcellulose non hydrosoluble, de préférence une éthylcellulose comme par exemple EC-N 50 NF ®(Hercules) ou encore Ethocel® (Premium Dow). Ajoutée en proportions adéquates à la masse de polymère hydrosoluble, l'éthylcellulose a pour effet d'allonger significativement la période de dissolution de l'insert ophtalmique et, par conséquent, la période de relargage de la substance médicamenteuse. Fait surprenant, l'incorporation d'éthylcellulose dans le matériau polymère choisi conformément à l'invention, conduit malgré tout à la dissolution complète de l'insert.

Selon l'invention, la matrice en matériau polymère comprend un polymère bioadhésif, c'est à dire un polymère naturel ou synthétique capable d'interaction stable avec un substrat biologique, telle la muqueuse du cul-de-sac conjonctival par exemple. A titre de polymère biocompatible bioadhésif, on peut avantageusement utiliser un polymère du type acide carboxylique polyvinylique (carboxy vinylpolymère) ou encore certains polysaccharides ou dérivés de polysaccharides bioadhésifs comme par exemple les éthers de cellulose, la méthylhydroxyéthylcellulose (Benecel® ME Aqualon, Tylose® MH Hoechst) ou la méthylhydroxypropyl-cellulose (Benecel® MP Aqualon) par exemple. On peut également utiliser des carboxyméthylcelluloses sodiques comme Blanose® Type 7 ou 9 (Aqualon) ou Tylose® C (Hoechst) par exemple.

On utilise de préférence un acide carboxylique polyvinylique non neutralisé, de poids moléculaire compris entre 450'000 et 4'000'000 tel un Carbopol® 934 P, 980, 984, 954, (Goodrich) ou Noveon® AA1 (Goodrich). Cet emploi de matériau non neutralisé dans un insert ophtalmique est à première vue surprenant puisque qu'il est reconnu comme irritant: en effet, celui-ci s'utilise normalement sous forme de sel de sodium. Dispersé en proportions adéquates au sein de la masse de matériau polymère, ce type de polymère bioadhésif perd son caractère irritant et garantit un temps de séjour prolongé de l'insert. En outre, ce type de polymère assure le positionnement de l'insert à son emplacement initial, offrant ainsi toute la sécurité voulue.

Selon l'invention, du fait que les polymères énumérés ci-dessus sont choisis en fonction de leur caractère extrudable ou thermoformable, la méthode de préparation de l'insert est optimale, notamment pour ce qui concerne le mélange intime des constituants et l'incorporation de la substance médicamenteuse. Le mélange des ingrédients choisis et leur extrusion ou thermoformage subséquents peuvent être effectués au moyens des techniques usuelles.

Dans une mise en oeuvre préférentielle de l'invention, les constituants de la matrice en matériau polymère composite se répartissent comme suit :
- hydroxypropylcellulose de 50 à 99,5 % en poids
- acide carboxylique polyvinylique non neutralisé de 0,5 à 5,0 % en poids,
   le complément éventuel à 100 % en poids étant constitué par de l'éthylcellulose.

Selon l'invention, on incorpore le plus généralement de 0,5 à 50 % poids, par exemple env. 25 % poids, de substance médicamenteuse dans l'insert en fonction de la nature de la substance médicamenteuse, du type d'affection à traiter et de l'effet recherché. L'insert selon l'invention se présentera le plus généralement sous forme de bâtonnet, de disque, pastille ou film en fonction des besoins.

A titre de substance médicamenteuse, on peut utiliser les agents appropriés les plus divers, tels des agents antibactériens, antiviraux, antimycotiques, des antiglaucomes, antiphlogistiques, antiinflammatoires, antiallergiques, vasoconstricteurs, vasodilatateurs, myotiques, mydriatiques, anesthésiants ou encore des préparations à action lubrifiante (larmes artificielles). Cette énumération n'est cependant pas exhaustive. L'invention sera illustrée à l'aide des exemples ci-après, qui ne sont en aucun cas limitatifs.

### Exemple 1

On prépare un insert contenant du sulfate de gentamycine comme substance médicamenteuse, incorporé à raison de 25 % poids dans une matrice en matériau polymère binaire obtenue à partir des composants suivants :
- hydroxypropylcellulose (Klucel® HXF - Aqualon)
- acide carboxylique polyvinylique non neutralisé (Carbopol® 934 P - Goodrich),
   à laquelle on ajoute encore env. 0,5 % (par rapport au poids total) de fluoréscéine sodique comme traceur UV.

Les échantillons sont préparés après mélange intime des ingrédients ci-dessus à l'état pulvérulent, puis extrusion à l'aide d'une extrudeuse à piston, dans les conditions suivantes température d'extrusion env. 160° C, durée 2 min, pression d'extrusion 200 kPa, diamètre de la filière 1,5 mm. Afin d'obtenir une homogénéisation parfaite des divers constituants, on procède à deux extrusions successives. Les inserts retenus pour expérimentation se présentent sous forme de bâtonnets de 5 mm de long et 1,35 à 1,45 mm de diamètre.

Pour l'expérimentation in vivo, les échantillons ont été déposés dans la partie postérieure du cul-de-sac conjonctival de lapins (1 à 5 sujets par expérimentation). La présence ou l'absence de l'insert est alors déterminée au moyen d'une lampe UV, des contrôles visuels étant effectués à intervalles réguliers. Les résultats obtenus ont été rassemblés dans le tableau ci-dessous.

| **Echant. no** | **HPC******* **%** | **Carbopol® %** | **Durée h** | **Taux d'échec** ****** |
|---|---|---|---|---|
| 011 | 100 | 0 | 7 | 40 |
| 013 | 99,5 | 0,5 | 8 | 0 |
| 018 | 99,0 | 1,0 | 16 | 0 |

| | | | | |
|---|---|---|---|---|
| * Hydroxypropylcellulose | | | | |
| **nombre d'inserts expulsés exprimé en % | | | | |

### Exemple 2

On prépare un insert contenant du sulfate de gentamycine commme substance médicamenteuse, incorporé à raison de 25 % poids dans une matrice en matériau polymère ternaire obtenue à partir des composants suivants :
- éthylcellulose EC N-50 NF®(Hercules)
- hydroxypropylcellulose (Klucel® HXF - Aqualon)
- acide carboxylique polyvinylique non neutralisé (Carbopol® 934 P - Goodrich),
   à laquelle on ajoute encore env. 0,5 % (par rapport ou poids total) de fluoréscéine sodique comme traceur UV .

Les échantillons sont ensuite soumis au processus d'extrusion décrit plus haut, pour être ensuite testés in vivo dans des conditions identiques, sur le lapin. Les résultats observés sont rassemblés dans le tableau ci-dessous.

| **Echant. no** | **HPC******* **%** | **EC******** **%** | **Carbopol® %** | **Durée h** | **Taux d'échec** ******* |
|---|---|---|---|---|---|
| 017 | 89,5 | 10,0 | 0,5 | 9 | 15 |
| 020 | 89,0 | 10,0 | 1,0 | 21 | 0 |
| 022 | 79,0 | 20,0 | 1,0 | 19 | 0 |
| 026 | 78,0 | 20,0 | 2,0 | 19 | 0 |
| 028 | 77,0 | 20,0 | 3,0 | 20 | 0 |
| 030 | 76,0 | 20,0 | 4,0 | 20 | 0 |
| 031 | 75,5 | 20,0 | 4,5 | 19 | 0 |

| | | | | | |
|---|---|---|---|---|---|
| * Hydroxypropylcellulose | | | | | |
| ** Ethylcellulose | | | | | |
| *** nombre d'inserts expulsés exprimés en % | | | | | |

## Revendications

1. Insert ophtalmique, en particulier insert ophtalmique destiné à la libération prolongée et contrôlée d'au moins une substance médicamenteuse, qui se compose d'une matrice en matériau polymère composite dans laquelle est incorporée la substance médicamenteuse, caractérisé en ce que ladite matrice en matériau polymère composite comprend, en mélange :
- un polymère biocompatible hydrosoluble et
- un polymère biocompatible bioadhésif,
ces deux polymères étant thermoformables ou extrudables.

2. Insert ophtalmique, en particulier insert ophtalmique destiné à la libération prolongée et contrôlée d'au moins une substance médicamenteuse, qui se compose d'une matrice en matériau polymère composite dans laquelle est incorporée la substance médicamenteuse, caractérisé en ce que ladite matrice en matériau polymère composite comprend, en mélange :
- un polymère biocompatible non hydrosoluble,
- un polymère biocompatible hydrosoluble et
- un polymère biocompatible bioadhésif,
ces trois polymères étant thermoformables ou extrudables.

3. Insert ophtalmique selon la revendication 1 ou 2, caractérisé en ce que la matrice en matériau polymère composite comprend de 50 à 99,5 % en poids du polymère biocompatible hydrosoluble et de 0,5 à 5 % en poids du polymère biocompatible bioadhésif, le complément éventuel à 100 % en poids étant constitué par le polymère biocompatible non-hydrosoluble.

4. Insert ophtalmique selon l'une des revendications 1 à 3, caractérisé en ce que le polymère biocompatible hydrosoluble est choisi parmi les hydroxyalkylcelluloses, les maltodextrines, les chitosans, les amidons modifiés tels les amidons destructurés ou les amidons partiellement hydrolysés, et les alcools polyvinyliques.

5. Insert ophtalmique selon la revendication 4, caractérisé en ce que l'hydroxyalkylcellulose est une hydroxypropylcellulose ayant un poids moléculaire compris entre 10'000 et 1'000'000.

6. Insert ophtalmique selon l'une des revendications 1 à 5, caractérisé en ce que le polymère biocompatible bioadhésif est choisi parmi les acides carboxyliques polyvinyliques et les polysaccharides et dérivés de polysaccharides bioadhésifs, de préférence un acide carboxylique polyvinylique non neutralisé ayant un poids moléculaire compris entre 450'000 et 4'000'000.

7. Insert ophtalmique selon l'une des revendications 2 à 6, caractérisé en ce que le polymère biocompatible non hydrosoluble est choisi parmi les alkylcelluloses non hydrosolubles.

8. Insert ophtalmique selon l'une des revendications 1 à 7, caractérisé en ce que la matrice en matériau polymère composite comprend de 50 à 99,5 % en poids d'hydropropylcellulose et de 0,5 à 5,0 % en poids d'acide carboxylique polyvinylique non neutralisé, le complément éventuel à 100 % étant constitué par de l'éthylcellulose.

9. Insert ophtalmique selon l'une des revendications 1 à 8, caractérisé en ce qu'il comporte de 0,5 à 50 % poids de substance médicamenteuse.

10. Insert ophtalmique selon l'une des revendications 1 à 9, caractérisé en ce que la substance médicamenteuse est choisie parmi les agents antibactériens, antiviraux, antimycotiques, antiglaucomes, antiphlogistiques, antiinflammatoires, antiallergiques, vasoconstricteurs, vasodilatateurs, myotiques, mydriatiques, anesthésiants et lubrifiants.

## Claims

1. An ophthalmic insert, more particularly an ophthalmic insert intended for the prolonged and controlled release of at least one medicinal substance, which comprises a matrix of composite polymeric material in which the medicinal substance is incorporated, characterized in that said composite polymeric material matrix comprises, as a mixture :
- a water-soluble biocompatible polymer and
- a bioadhesive biocompatible polymer,
said two polymers being extrudable or thermoformable.

2. An ophthalmic insert, more particularly an ophthalmic insert intended for the prolonged and controlled release of at least one medicinal substance, which comprises a matrix of composite polymeric material in which the medicinal substance is incorporated, characterized in that said composite polymeric material matrix comprises, as a mixture :
- a water-insoluble biocompatible polymer,
- a water-soluble biocompatible polymer and
- a bioadhesive biocompatible polymer,
these three polymers being extrudable or thermoformable.

3. An ophthalmic insert according to claim 1 or 2, characterised in that the composite polymeric material matrix comprises from 50 to 99.5% by weight of the water-soluble biocompatible polymer and from 0.5 to 5% by weight of the bioadhesive biocompatible polymer, any remainder up to 100% by weight being the water-insoluble biocompatible polymer.

4. An ophthalmic insert according to any one of claims 1 to 3, characterized in that the water-soluble biocompatible polymer is selected from the hydroxyalkyl celluloses, maltodextrins, chitosans, modified starches such as destructured or partially hydrolysed starches, and polyvinyl alcohols.

5. An ophthalmic insert according to claim 4, characterized in that the hydroxyalkyl cellulose is a hydroxypropyl cellulose having a molecular weight of between 10 000 and 1 000 000.

6. An ophthalmic insert according to any one of claims 1 to 5, characterized in that the bioadhesive biocompatible polymer is selected from the polyvinyl carboxylic acids and bioadhesive polysaccharides or polysaccharide derivatives, preferably a non-neutralised polyvinyl carboxylic acid having a molecular weight of between 450 000 and 4 000 000.

7. An ophthalmic insert according to any one of claims 2 to 6, characterized in that the water- insoluble biocompatible polymer is selected from the water-insoluble alkyl celluloses.

8. An ophthalmic insert according to any one of claims 1 to 7, characterized in that the composite polymeric material matrix comprises from 50 to 99.5% by weight of hydroxypropyl cellulose and from 0.5 to 5.0% by weight of non-neutralised polyvinyl carboxylic acid, any remainder up to 100% being ethyl cellulose.

9. An ophthalmic insert according to any one of claims 1 to 8, characterized in that it comprises 0.5 to 50% by weight of medicinal substance.

10. An ophthalmic insert according to any one of claims 1 to 9, characterized in that the medicinal substance is selected from amongst antibacterial, antiviral, antimycotic, anti-glaucoma, antiphlogistic, anti-inflammatory, anti-allergy, vasoconstrictive, vaso-dilatory, myotic, mydriatic, anaesthetic and lubricant agents.

## Patentansprüche

1. Augen-Einsatz, insbesondere zur langfristigen und kontrollierten Freisetzung mindestens einer medikamentösen Substanz bestimmter Augen-Einsatz, bestehend aus einer Matrix aus zusammengesetztem Polymermaterial, in welche die medikamentöse Substanz eingebaut ist, dadurch gekennzeichnet, daß genannte Matrix aus zusammengesetztem Polymermaterial in Mischung enthält:
- ein wasserlösliches bioverträgliches Polymer und
- ein bioadhäsives bioverträgliches Polymer,
wobei diese beiden Polymere wärmeverformbar oder extrudierbar sind.

2. Augen-Einsatz, insbesondere zur langfristigen und kontrollierten Freisetzung mindestens einer medikamentösen Substanz bestimmter Augen-Einsatz, bestehend aus einer Matrix aus zusammengesetztem Polymermaterial, in welche die medikamentöse Substanz eingebaut ist, dadurch gekennzeichnet, daß genannte Matrix aus zusammengesetztem Polymermaterial in Mischung enthält:
- ein wasserunlösliches bioverträgliches Polymer,
- ein wasserlösliches bioverträgliches Polymer und
- ein bioadhäsives bioverträgliches Polymer,
wobei diese drei Polymere wärmeverformbar oder extrudierbar sind.

3. Augen-Einsatz nach Anspruch 1 oder 2, dadurch gekennzeichnet, daß die Matrix aus zusammengesetztem Polymermaterial 50 bis 99,5 Gew.-% des wasserlöslichen bioverträglichen Polymers und 0,5 bis 5 Gew.-% des bioadhäsiven bioverträglichen Polymers enthält, wobei ein etwaiger bis zu 100 Gew.-% fehlender Rest aus dem wasserunlöslichen bioverträglichen Polymer besteht.

4. Augen-Einsatz nach einem der Ansprüche 1 bis 3, dadurch gekennzeichnet, daß das wasserlösliche bioverträgliche Polymer unter den Hydroxyalkylcellulosen, den Maltodextrinen, den Chitosanen, den modifizierten Stärken, etwa den abgebauten Stärken oder den teilweise hydrolysierten Stärken, und den Polyvinylalkoholen gewählt ist.

5. Augen-Einsatz nach Anspruch 4, dadurch gekennzeichnet, daß die Hydroxyalkylcellulose eine Hydroxypropylcellulose ist, die ein Molekulargewicht zwischen 10 000 und 1 000 000 hat.

6. Augen-Einsatz nach einem der Ansprüche 1 bis 5, dadurch gekennzeichnet, daß das bioadhäsive bioverträgliche Polymer unter den Polyvinylcarbonsäuren und den bioadhäsiven Polysacchariden und Polysaccharidderivaten gewählt ist, vorzugsweise eine nicht neutralisierte Polyvinylcarbonsäure ist, die ein Molekulargewicht zwischen 450 000 und 4 000 000 hat.

7. Augen-Einsatz nach einem der Ansprüche 2 bis 6, dadurch gekennzeichnet, daß das wasserunlösliche bioverträgliche Polymer unter den wasserunlöslichen Alkylcellulosen gewählt ist.

8. Augen-Einsatz nach einem der Ansprüche 1 bis 7, dadurch gekennzeichnet, daß die Matrix aus zusammengesetztem Polymermaterial 50 bis 99,5 Gew.-% Hydroxypropylcellulose und 0,5 bis 5,0 Gew.-% nicht neutralisierte Polyvinylcarbonsäure enthält, wobei ein etwaiger bis zu 100 Gew.-% fehlender Rest aus Ethylcellulose besteht.

9. Augen-Einsatz nach einem der Ansprüche 1 bis 8, dadurch gekennzeichnet, daß es 0,5 bis 50 Gew.-% medikamentöse Substanz enthält.

10. Augen-Einsatz nach einem der Ansprüche 1 bis 9, dadurch gekennzeichnet, daß die medikamentöse Substanz unter den antibakteriellen, antiviralen, antimykotischen, gegen Glaukome gerichteten, antiphlogistischen, antiinflammatorischen, antiallergischen, vasokonstriktorischen, vasodilatatorischen, miotischen, mydriatischen, anästhetisierenden und lubrifizierenden Wirkstoffen gewählt ist.
